# EUROPÄISCHE PATENTANMELDUNG

(11) **EP 0 812 569 A1**
(43) Veröffentlichungstag der Anmeldung: **17.12.1997**
(21) Anmeldenummer: 96109249.1
(22) Anmeldetag: 10.06.1996
(51) Int. Cl.: A61B 10/00

(54) **Verfahren zur Bestimmung der Bioverfügbarkeit und/oder Blut-Hirn-Schrankenpermeabilität einer chemischen Verbindung**

(71) Anmelder: ARROWDEAN LTD., IE-Dublin 2 (IE)
(72) Erfinder: Mattern, Claudia, Dr., 82335 Starnberg (DE); Häcker, Rüdiger, Prof. Dr., 6600 Greiz (AT); Svoboda, Tomás, Dr., 141 00 Praha (CZ); Sedlák, Libor, Dr., 254 49 Jilové u Prahy (CZ)
(74) Vertreter: Winkler, Andreas, Dr.

(57) **Zusammenfassung**

Die Erfindung betrifft ein Verfahren zur Bestimmung der Bioverfügbarkeit und/oder Blut/Hirnschrankengängigkeit einer chemischen Verbindung oder Kombination von chemischen Verbindungen, welches dadurch gekennzeichnet ist, daß
a) eine Vorrichtung zur Entnahme von Cerebrospinalflüssigkeit in einen Cerebralventrikel eines Schafes inseriert wird;
b) die chemische Verbindung oder Kombination von chemischen Verbindungen dem Schaf appliziert wird;
c) Cerebrospinalflüssigkeit aus dem Cerebralventrikel entnommen wird; und
d) die Konzentration der chemischen Verbindung(en) in der dem Cerebralventrikel des Schafes entnommenen Cerebrospinalflüssigkeit bestimmt wird.

## Beschreibung

Die Erfindung betrifft ein Verfahren zur Bestimmung der Bioverfügbarkeit und/oder Blut/Hirnschrankengängigkeit einer chemischen Verbindung.

Kaum eine andere Struktur des menschlichen und tierischen Organismus hat von jeher den Menschen so in seinen Bann geschlagen wie das Gehirn. Entsprechend dem jeweiligen soziokulturellen Hintergrund war dabei die Beschäftigung mit dem Gehirn manchmal mehr naturwissenschaftlich oder mehr religiös-philosophisch geprägt. Diese Trennung mag zumindest für den westlichen Kulturkreis in der berühmten Äußerung Rene Descartes "cogito, ergo sum" begründet worden sein. In der Folge haben sich beide Richtungen fast unabhängig voneinander weiterentwickelt, um sich erst in der jüngsten Vergangenheit wieder einander anzunähern, wobei der naturwissenschaftliche Aspekt der Klärung von Fragestellungen, die früher ausschließlich der Philosophie vorbehalten waren, wie z.B. die des Bewußtseins, an Einfluß gewinnt.

Neben den erkenntnistheoretischen Beweggründen spielen zumindest in jüngster Vergangenheit auch solche aus dem Bereich der Medizin eine immer größere Rolle, wenn es gilt, die der Funktion des Gehirns zugrundeliegenden Mechanismen zu untersuchen und zu verstehen.

Bedingt durch die insgesamt besseren Lebensbedingungen ist in den vergangenen Jahrhunderten, und speziell den letzten Jahrzehnten, die durchschnittliche Lebenserwartung deutlich gestiegen. In der Folge kam es zum Auftreten von Krankheitsbildern, die aufgrund einer früher anders verlaufenden demographischen Entwicklung der Bevölkerung als solche nicht oder nur vergleichsweise selten beobachtet wurden. Darüber hinaus scheint auch das Vordringen neuer, bisher als solches nicht faßbarer oder in der medizinischen Literatur nicht beschriebenen degenerativen Hirnleiden, die nicht als direktes oder indirektes Resultat des Alterns neuronaler Strukturen verstanden werden können, für eine zunehmende Häufung von neurologischen Erkrankungen im weitesten Sinne verantwortlich zu sein.

Anders als vor nicht allzu langer Zeit, in der Menschen mit neurologischen Befunden von der Gesellschaft ausgegrenzt wurden, sei es nun in Form einer gesellschaftlichen Ächtung oder, wenngleich nicht im europäischen Kulturkreis, in Form einer sakralen Überhebung als Ausdruck der Nähe zu einem höheren Wesen, steht heute die Bewahrung eines Bewußtseinszustandes im Vordergrund, der eine Orientierung des von einem derartigen Leiden Betroffenen in seinem räumlichen und sozialen Umfeld erlaubt. Entsprechend intensiv wird an der Entwicklung von Medikamenten zur Behandlung derartiger neurologischer Erkrankungen gearbeitet, von denen hier als Beispiel nur die Parkinsonsche Krankheit und die Alzheimersche Krankheit angeführt seien.

Ein besonders wichtiger Teilaspekt dieser Arbeit ist darin zu sehen, daß es erforderlich ist, die für die Therapie solcher Erkrankungen vorgesehenen Wirkstoffe so zu gestalten, entweder direkt oder durch Verwendung geeigneter Darreichungsformen, daß sie an jene Stellen des Gehirns bzw. zentralen Nervensystems gelangen, die ihren bestimmungsgemäßen Wirkort darstellen. Dabei gilt es zu berücksichtigen, daß zwar die Gefäßkapillaren für Pharmaka in der Regel gut durchlässig sind, jedoch die Kapillaren im zentralen Nervensystem hiervon eine Ausnahme bilden. Letztere sind von einer dichten Gliazellschicht umgeben, die sich wie eine Lipidbarriere verhält und für die Bioverfügbarkeit und Blut/Hirnschrankengängigkeit von chemischen Verbindungen von zentraler Bedeutung ist.

Gegenwärtig gibt es keine verläßliche Methode, diese Bioverfügbarkeit im Zentralnervensystem unter physiologischen Bedingungen zu bestimmen und daraus Schlußfolgerungen für eine Optimierung der Therapie abzuleiten. Die Pharmakokinetik von Wirkstoffen im Blutkreislauf erlaubt aus den oben angeführten Gründen keine sichere Aussage zur Bioverfügbarkeit im Gehirn.

Für Messungen an Tiermodellen, vorzugsweise Ratten, sind Verfahren entwickelt worden, bei denen bestimmte Hirnabschnitte kanuliert werden. Diese Methoden erfordern anästhesierte Tiere, die Probenentnahme ist auf sehr geringe Mengen beschränkt und jedes Tier muß nach Durchführung des Versuches getötet werden. Reproduzierbare Ergebnisse und verallgemeinerungsfähige Aussagen können daher nur mit einem sehr großen Arbeits- und vor allem Tieraufwand erreicht werden.

Darüber hinaus ist zu berücksichtigen, daß die Ratte zwar eines der gebräuchlichsten Versuchstiere für pharmakologisch-pharmazeutische Fragestellungen ist, jedoch ihr Stoffwechsel, insbesondere was den Metabolismus von pharmakologischen wirksamen chemischen Verbindungen betrifft, in wichtigen Reaktionsabläufen gravierende Unterschiede zum Menschen aufweist. Weiterhin führt der Aufbau der genannten Versuchsanordnungen zwangsläufig dazu, daß unphysiologische Verhältnisse und teilweise Schädigungen der Hirnsubstanz in Kauf genommen werden müssen. Die Anwendung bildgebender Verfahren, wie der PET (Positronemissionstomographie) behebt diese Mängel nicht, da dieses Verfahren zwar halbquantitative Aussagen erlaubt, eine selektive Erfassung der Konzentration und der chemischen Natur der untersuchten Wirkstoffe aber nicht möglich sind.

Damit ist eine übertragbarkeit der Ergebnisse zur Bioverfügbarkeit und/oder Blut/Hirnschrankengängigkeit von zentralnerval wirksamen chemischen Verbindungen, die an den zur Zeit in der Literatur beschriebenen Tiermodellen gewonnen werden können, auf den Menschen nur eingeschränkt oder überhaupt nicht möglich.

Der Erfindung liegt die Aufgabe zugrunde, ein einfaches und zuverlässiges Verfahren zur Bestimmung der Bioverfügbarkeit und/oder Blut/Hirnschrankengängigkeit einer chemischen Verbindung oder Kombination von chemischen Verbindungen bereitzustellen. Eine weitere Aufgabe ist darin zu sehen, daß eine derartige Bestimmung der Bioverfügbarkeit und/oder Blut/Hirnschrankengängigkeit unter weitestgehend physiologischen Bedingungen vorgenommen werden kann. Weiterhin umfaßt die Aufgabe, daß die durch das bereitzustellende Verfahren bestimmte Bioverfügbarkeit und/oder Blut/Hirnschrankengängigkeit leicht und zutreffend auf die Verhältnisse beim Menschen übertragen werden kann. Schließlich soll das Verfahren eine hohe Reproduzierbarkeit gewährleisten und kostengünstig sein.

Erfindungsgemäß wird die Aufgabe gelöst durch ein Verfahren zur Bestimmung der Bioverfügbarkeit und/oder Blut/Hirnschrankengängigkeit einer chemischen Verbindung oder Kombination von chemischen Verbindungen, dadurch gekennzeichnet, daß:
a) eine Vorrichtung zur Entnahme von Cerebrospinalflüssigkeit in einen Cerebralventrikel eines Schafes inseriert wird;
b) die chemische Verbindung oder Kombination von chemischen Verbindungen dem Schaf appliziert wird;
c) Cerebrospinalflüssigkeit aus dem Cerebralventrikel entnommen wird; und
d) die Konzentration der chemischen Verbindung(en) in der dem Cerebralventrikel des Schafes entnommenen Cerebrospinalflüssigkeit bestimmt wird.

In einer Ausführungsform kann vorgesehen sein, das auch vor Schritt b) Cerebrospinalflüssigkeit aus dem Cerebralventrikel entnommen wird.

Die Erfindung schlägt vor, daß der Cerebralventrikel der dritte Cerebralventrikel ist.

Es kann vorgesehen sein, daß die Vorrichtung zur Entnahme von Cerebrospinalflüssigkeit eine Kanüle ist.

In einer weiteren Ausführungsform der Erfindung ist vorgesehen, daß die Vorrichtung zur Entnahme von Cerebrospinalflüssigkeit stereotaktisch in den Cerebralventrikel inseriert wird.

In einer bevorzugten Ausführungsform ist vorgesehen, daß die stereotaktische Insertion auf der Basis des Knochenreferenzsystems erfolgt.

In einer ganz besonders bevorzugten Ausführungsform ist vorgesehen, daß die stereotaktische Insertion zusätzlich auf der Basis geeigneter stereotaktischer Diagramme erfolgt.

Die Erfindung schlägt vor, daß die Schädeldecke an der Stelle, an der die Vorrichtung zur Entnahme von Cerebrospinalflüssigkeit inseriert werden soll, mittels gezielter Trepanation eröffnet wird.

Weiterhin schlägt die Erfindung vor, daß die Dura mater durch die Spitze der Vorrichtung zur Entnahme von Cerebrospinalflüssigkeit perforiert wird.

In einer Ausführungsform kann vorgesehen sein, daß die Vorrichtung zur Entnahme von Cerebrospinalflüssigkeit aus zwei konzentrischen Röhren besteht, wobei die äußere Röhre als Führung dient und die innere Röhre beweglich und für die Entnahme von Cerebrospinalflüssigkeit aus dem Cerebralventrikel bestimmt ist.

In einer bevorzugten Ausführungsform ist vorgesehen, daß die äußere Röhre der Kanüle in der Trepanationsöffnung fixiert wird.

In einer ganz besonders bevorzugten Ausführungsfom ist vorgesehen, daß die äußere Röhre der Kanüle mit Acrylatharz fixiert wird.

Die Erfindung schlägt des weiteren vor, daß die chemische Verbindung oder Kombination von chemischen Verbindungen mindestens eine pharmakologisch wirksame Substanz umfaßt.

In einer Ausführungsforn des erfindungsgemäßen Verfahrens kann auch vorgesehen sein, daß die chemische Verbindung oder Kombination von chemischen Verbindungen eine galenische Darreichungsform umfaßt.

Die Erfindung schlägt vor, daß die chemische Verbindung oder Kombination von chemischen Verbindungen einmalig appliziert wird.

In einer Ausführungsform des erfindungsgemäßen Verfahrens kann vorgesehen sein, daß die chemische Verbindung oder Kombination von chemischen Verbindungen mehrfach appliziert wird.

In einer weiteren Ausführungsform kann vorgesehen sein, daß die chemische Verbindung oder Kombination von chemischen Verbindungen als Depot appliziert wird.

In einer weiteren Ausführungsform des erfindungsgemäßen Verfahrens tritt Cerebrospinalflüssigkeit spontan aus der Vorrichtung zur Entnahme von Cerebrospinalflüssigkeit aus.

In einer anderen Ausführungsform des erfindungsgemäßen Verfahrens ist vorgesehen, daß Cerebrospinalflüssigkeit spontan aus der Vorrichtung zur Entnahme von Cerebrospinalflüssigkeit nach Entnahme eines Verschlußmittels aus der besagten Vorrichtung austritt.

Die Erfindung schlägt vor, daß die Cerebrospinalflüssigkeit abgesaugt wird. In einer bevorzugten Ausführungsform wird die Cerebrospinalflüssigkeit mit einer Spritze abgesaugt.

Weiterhin schlägt die Erfindung vor, daß in zeitlichen Abständen mehrfach hintereinander Cerebrospinalflüssigkeit entnommen wird.

In einer besonders bevorzugten Ausführungsform ist dabei vorgesehen, daß die Entnahme von Cerebrospinalflüssigkeit in Abständen von etwa 15 Minuten erfolgt.

Bei dem erfindungsgemäßen Verfahren kann vorgesehen sein, daß pro Entnahme von Cerebrospinalflüssigkeit etwa 0,3 bis etwa 0,6 ml Cerebrospinalflüssigkeit entnommen werden.

Die Erfindung schlägt des weiteren vor, daß die Vorrichtung zur Entnahme von Cerebrospinalflüssigkeit aus einem Material hergestellt ist, das aus der Gruppe ausgewählt ist, die rostfreien Stahl und Teflon umfaßt.

Weiterhin schlägt die Erfindung vor, daß die Vorrichtung zur Entnahme von Cerebrospinalflüssigkeit aus der Gruppe ausgewählt ist, die kommerziell erhältliche Injektions- und Punktionskanülen umfaßt.

In einer Alternative des erfindungsgemäßen Verfahrens ist vorgesehen, daß nach der letzten Entnahme von Cerebrospinalflüssigkeit die Vorrichtung zur Entnahme von Cerebrospinalflüssigkeit aus dem Cerebralventrikel entfernt wird.

In einer weiteren Alternative des erfindungsgemäßen Verfahrens kann vorgesehen sein, daß nach der letzten Entnahme von Cerebrospinalflüssigkeit die Vorrichtung zur Entnahme von Cerebrospinalflüssigkeit im Cerebralventrikel verbleibt.

In einer Ausführungsform des erfindungsgemäßen Verfahrens kann vorgesehen sein, daß das Schaf während der Öffnung der Schädeldecke betäubt ist.

Weiterhin kann bei dem erfindungsgemäßen Verfahren vorgesehen sein, daß das Schaf nach Applikation der chemischen Verbindung(en) frei laufen gelassen wird.

Der vorliegenden Erfindung liegt die überraschende Erkenntnis zugrunde, daß bei einem Verfahren zur Bestimmung der Bioverfügbarkeit und/oder Blut/Hirnschrankengängigkeit einer chemischen Verbindung oder Kombination von chemischen Verbindungen, bei dem
a) eine Vorrichtung zur Entnahme von Cerebrospinalflüssigkeit in einen Cerebralventrikel eines Schafes inseriert wird;
b) die chemische Verbindung oder Kombination von chemischen Verbindungen dem Schaf appliziert wird;
c) Cerebrospinalflüssigkeit aus dem Cerebralventrikel entnommen wird; und
d) die Konzentration der chemischen Verbindung(en) in der dem Cerebralventrikel des Schafes entnommenen Cerebrospinalflüssigkeit bestimmt wird;
auf einfache und zuverlässige Art und Weise die Bioverfügbarkeit und/oder Blut/Hirnschrankengängigkeit einer chemischen Verbindung oder Kombination von chemischen Verbindungen bestimmt werden kann.

Weiterhin überraschend war in diesem Zusammenhang die Erkenntnis, daß diese unter Verwendung des erfindungsgemäßen Verfahrens gewonnenen Werte zur Bioverfügbarkeit und/oder Blut/Hirnschrankengängigkeit leicht und zutreffend auf die Verhältnisse beim Menschen übertragen werden können.

Ein Vorteil des erfindungsgemäßen Verfahrens ist darin zu sehen, daß sowohl die neuroanatomischen Verhältnisse als auch besonders der Metabolismus von Wirkstoffen beim Schaf eine hohe Vergleichbarkeit zu den Verhältnissen beim Menschen aufweisen.

Ein weiterer Vorteil des erfindungsgemäßen Verfahrens ist darin zu sehen, daß die durch das erfindungsgemäße Verfahren erhaltenen Werte zur Bioverfügbarkeit und/oder Blut/Hirnschrankengängigkeit unter weitestgehend physiologischen Bedingungen erhalten werden können.

Der Vorteil der Verwendung eines Schafes beim erfindungsgemäßen Verfahren liegt auch darin begründet, daß mit einem Schaf, als landwirtschaftliches Nutztier, entsprechende Studien an einer Vielzahl von leicht erhältlichen und leicht zu haltenden Tieren möglich ist.

Schließlich erlaubt das erfindungsgemäße Verfahren auch einen hohen Grad an Reproduzierbarkeit, was sowohl durch die geringe Anzahl von vorzunehmenden Verfahrensschritten bedingt wird als auch durch die Tatsache, daß ein Tier mehrfach zu Untersuchungen verwendet werden kann und somit eine direkte Vergleichbarkeit der Ergebnisse gegeben ist.

Wird bei dem erfindungsgemäßen Verfahren vor Schritt b) Cerebrospinalflüssigkeit aus dem Cerebralventrikel entnommen, ergibt sich damit die Möglichkeit, einen exakt definierten Referenzwert oder Nullprobe für die Bestimmung der Bioverfügbarkeit und/oder Blut/Hirnschrankengängigkeit einer chemischen Verbindung oder Kombination von chemischen Verbindungen zu erhalten. Dieser Aspekt ist besonders bei Verbindungen von Bedeutung, die entweder unter physiologischen Bedingungen bereits in geringen Konzentrationen im Zentralnervensystem vorhanden sind oder schnell aus den Blutgefäßen in die Cerebrospinalflüssigkeit übertreten, da bevorzugt unter diesen Verfahrensbedingungen eine exakte Auflösung der für die Kinetik maßgeblichen Anfangszeiten relevant ist. Neben der Ermittlung der besagten Werte selbst können damit auch Rückschlüsse auf die unter Umständen an der Passage beteiligten Transportsysteme gezogen werden, was für die weitere Entwicklung der Verbindung oder ggf. auch entsprechender Inhibitoren, die zu einer Optimierung einer Therapie führen können, von großem Interesse ist.

Der dritte Cerebralventrikel ist für das erfindungsgemäße Verfahren insoweit von besonderer Bedeutung, als daß er vergleichsweise leicht zugänglich ist und - auch bedingt durch sein Volumen - leicht punktiert werden kann, was mit Blick auf die Tatsache, daß es zwischen den einzelnen Schafen betreffend ihrer neuroanatomischen Struktur im Zentralnervensystem durchaus zu Unterschieden kommen kann, von besonderer Relevanz ist.

Weiterhin befinden sich am Boden des dritten Cerebralventrikels und in seiner unmittelbaren Nachbarschaft neuroanatomische Strukturen und funktionelle Regelkreise, die wichtige Körperfunktionen beeinflussen und demzufolge auch Zielorte von Pharmaka sind, die eine Wirkung im Zentralnervensystem entfalten sollen.

Darüber hinaus erlaubt die Größe des dritten Cerebralventrikels auch die mehrfache Entnahme von Cerebrospinalflüssigkeit innerhalb kurzer Zeitabstände.

Wird im erfindungsgemäßen Verfahren eine Kanüle als Vorrichtung zur Entnahme von Cerebrospinalflüssigkeit verwendet, ist eine weitere Begrenzung der Kosten möglich und darüber hinaus auch gewährleistet, daß selbst unter Bedingungen, in denen nur ein vergleichsweise geringes Instrumentarium zur Verfügung steht, die genannten Untersuchungen durchgeführt werden können. Darüber hinaus gewährleistet die Verwendung einer Kanüle, sei es einer Einwegkanüle oder einer Mehrfachkanüle, daß die Sterilität der besagten Vorrichtung zur Entnahme von Cerebrospinalflüssigkeit zuverlässig realisiert werden kann, was letztenendes dazu führt, daß die Versuchsergebnisse beispielsweise nicht dadurch beeinträchtigt werden, daß es infolge des Verfahrens zu einer Infektion kommt und somit der physiologische Normalzustand des Schafes nicht gegeben wäre.

Die stereotaktische Insertion der Vorrichtung zur Entnahme von Cerebrospinalflüssigkeit in den Cerebralventrikel bedingt, daß nur eine vergleichsweise geringe Beschädigung der Hirnstrukturen, wenn überhaupt, auftritt. Typischerweise basiert das im Rahmen des erfindungsgemäßen Verfahrens zur Anwendung gelangende stereotaktische Verfahren auf einem Knochenreferenzsystem.

Die genannten Vorteile des Verfahrens zur stereotaktischen Insertion auf der Basis des Knochenreferenzsystems werden noch dadurch erhöht, daß dieses zusätzlich auf der Basis geeigneter stereotaktischer Diagramme erfolgt.

Um die durch den Eingriff am Tier bedingten Störungen möglichst gering zu halten, aus Tierschutzgründen ebenso wie aus Gründen der optimalen Ermittlung der Werte zur Bioverfügbarkeit und/oder Blut/Hirnschrankengängigkeit, sollte die Schädeldecke an der Stelle, an der die Vorrichtung zur Entnahme von Cerebrospinalflüssigkeit inseriert werden soll, mittels gezielter Trepanation eröffnet werden.

Aufgrund der bedeutenden Funktion der Hirnhäute, und im speziellen der Dura mater, ist es auch hier aus den obengenannten Gründen besonders vorteilhaft, die zwangsläufig mit dem Eingriff verbundenen Läsionen möglichst gering zu halten. Ein derartiger minimaler Eingriff wird dadurch möglich, daß die Hirnhäute und besonders die Dura mater durch die Spitze der Vorrichtung zur Entnahme von Cerebrospinalflüssigkeit perforiert werden und solchermaßen dem angestrebten Zustand der Aufrechterhaltung weitestgehend physiologischer Bedingungen im Rahmen des erfindungsgemäßen Verfahrens weiter nachgekommen wird.

Die Gestaltung der im erfindungsgemäßen Verfahren verwendeten Vorrichtung zur Entnahme von Cerebrospinalflüssigkeit dient ebenfalls der Begrenzung der durch den Eingriff unvermeidlichen Störung des Gesamtsystems. Besteht die Vorrichtung aus zwei konzentrischen Röhren, wobei die äußere Röhre als Führung dient und die innere Röhre beweglich und für die Entnahme von Cerebrospinalflüssigkeit aus dem Cerebralventrikel bestimmt ist, wird eine funktionale Trennung von Einführung und Fixierung einerseits und Entnahme von Cerebrospinalflüssigkeit aus dem Cerebralventrikel andererseits gewährleistet. Dies bedeutet, daß nach einmaliger Insertion das neuronale System des Schafes von weiteren Störungen nur mittelbar betroffen ist, besonders dann, wenn die äußere Röhre der Kanüle in der Trepanationsöffnung fixiert wird. Somit kann auch unter den Bedingungen eines Verfahrens, in dem sich das Versuchstier nach Insertion der Vorrichtung zur Entnahme von Cerebrospinalflüssigkeit in einen Cerebralventrikel frei bewegen kann, sich die Kanüle bzw. Entnahmevorrichtung nicht aus ihrer erwünschten und definierten Position lösen. Somit werden die nachfolgende bzw. nachfolgenden Entnahmen zuverlässig möglich und eine weitergehende, unnötige Zerstörung von Gehirnstrukturen vermieden.

Als besonders vorteilhaft hat sich für die Fixierung der äußeren Röhre der Kanüle die Verwendung von Acrylatharzen erwiesen.

Das erfindungsgemäße Verfahren kann besonders dann vorteilhaft eingesetzt werden, wenn die chemische Verbindung oder Kombination von chemischen Verbindungen mindestens eine pharmakologisch wirksame Substanz umfaßt.

Die Kenntnis derartiger Daten zur Bioverfügbarkeit und/oder Blut/Hirnschrankengängigkeit ist für die Entwicklung von geeigneten Arzneimitteln zur Behandlung neurologischer Defekte, besonders im zentralen Nervensystem, von entscheidender Bedeutung, da alleine die Verfügbarkeit bzw. Konzentration der pharmakologisch wirksamen Substanz am Wirkort für den Erfolg einer derartigen Behandlung maßgeblich ist.

Das oben genannte Erfordernis der Verfügbarkeit entsprechender Daten ist auch und besonders dann gegeben, wenn die chemische Verbindung oder Kombination von chemischen Verbindungen in einer galenischen Darreichungsform vorliegt.

Das erfindungsgemäße Verfahren erlaubt auch die typischen Applikationsformen, wie sie bei der Therapie und Diagnose sowohl in der Human- als auch in der Veterinärmedizin eingesetzt werden, zu realisieren. So sieht das erfindungsgemäße Verfahren vor, daß die chemische Verbindung oder Kombination von chemischen Verbindungen einmalig appliziert wird.

Demzufolge können erstmalig die pharmakokinetischen Verhältnisse während und nach der Passage durch die Blut/Hirnschranke studiert werden, beispielsweise bei Beginn der Verabreichung der in Frage kommenden chemischen Verbindung oder Kombination von chemischen Verbindungen. Darüber hinaus erlaubt das erfindungsgemäße Verfahren auch, daß die chemische Verbindung oder Kombination von chemischen Verbindungen mehrfach appliziert oder als Depot appliziert wird. Somit können die entsprechenden Verhältnisse auch im Tiermodell simuliert werden und entsprechende Daten zur Verfügung gestellt werden betreffend die Bioverfügbarkeit und/oder Blut/Hirnschrankengängigkeit unter den genannten und weiteren Applikationsbedingungen, nämlich mehrfacher Applikation oder Applikation in Form eines Depots, wie sie z.B. bei chronisch Kranken oder Kranken, denen eine entsprechende Verbindung oder Kombination von Verbindungen über einen längeren Zeitraum verabreicht werden soll, üblich sind. Entsprechend lassen sich auch die unter derartigen Applikationsbedingungen auftretenden Probleme im erfindungsgemäßen Verfahren simulieren und somit letztendlich die erforderlichen Rückschlüsse für die Optimierung einer therapeutischen oder diagnostischen Anwendung beim Menschen oder Tier ziehen.

Tritt bei dem erfindungsgemäßen Verfahren Cerebrospinalflüssigkeit spontan aus der Vorrichtung zur Entnahme von Cerebrospinalflüssigkeit aus, sind die Voraussetzungen dafür gegeben, daß infolge des eigentlichen Entnahmevorganges keine weiteren, unter Umständen die Meßwerte verändernden Störungen auftreffen.

Gleiches gilt auch für den Fall, daß Cerebrospinalflüssigkeit spontan aus der Vorrichtung zur Entnahme der Cerebrospinalflüssigkeit nach Entfernung eines Verschlußmittels aus der besagten Vorrichtung austritt, wobei das besagte Verschlußmittel beispielsweise ein Mandrin oder eine sonstige Hemmvorrichtung ist. Auf diese Art und Weise wird sichergestellt, daß es zu keinem Verlust der Cerebrospinalflüssigkeit infolge spontanen Austritts aus der Vorrichtung zur Entnahme von Cerebrospinalflüssigkeit kommt. Dieser Aspekt ist besonders unter Bedingungen wichtig, in denen ein vergleichsweise langsamer Übertritt der chemischen Verbindung oder Kombination von chemischen Verbindungen, deren Bioverfügbarkeit und/oder Blut/Hirnschrankengängigkeit bestimmt werden soll, erfolgt.

Umgekehrt ermöglicht das Absaugen von Cerebrospinalflüssigkeit im Rahmen des erfindungsgemäßen Verfahrens, daß besonders bei hoher Bioverfügbarkeit und/oder Blut/Hirnschrankengängigkeit der chemischen Verbindung oder Kombination von chemischen Verbindungen die Möglichkeit geschaffen wird, die für die Bestimmung der besagten Werte notwendigen Proben innerhalb kürzester Zeit unabhängig von der spontanen Austrittsgeschwindigkeit zu erhalten.

Die Verwendung einer Spritze ist insoweit besonders angezeigt, als damit ein vergleichsweise einfaches und kostengünstiges Mittel zum Einsatz gelangen kann, um die oben erwähnten Vorteile zu erlangen.

Wird bei dem erfindungsgemäßen Verfahren Cerebrospinalflüssigkeit in zeitlichem Abstand mehrfach hintereinander entnommen, ist eine Bestimmung der kinetischen Daten betreffend die Bioverfügbarkeit und/oder Blut/Hirnschrankengängigkeit möglich, die genauere Rückschlüsse zuläßt als eine ausschließliche Bestimmung des Endpunktes oder eines Punktes nach einer definierten Zeit.

Zeitabstände betreffend die Entnahme von Cerebrospinalflüssigkeit von etwa 15 Minuten haben sich dabei als besonders vorteilhaft erwiesen. Diese Vorteile ergeben sich überwiegend aus der Tatsache, daß die typischerweise applizierte chemische Verbindung oder Kombination von chemischen Verbindungen signifikante Änderungen im Cerebrospinalflüssigkeitsspiegel in Zeiträumen der genannten Größenordnung bedingt. Ein weitergehender Vorteil dieser Zeitabstände ist darin zu sehen, daß dieser Zeitraum auch zu erlauben scheint, daß entsprechende, bei vorangehenden Entnahmen entnommene Cerebrospinalflüssigkeit zwischenzeitlich im Cerebralventrikel wieder auf physiologischem Wege ersetzt worden ist.

Andere Zeitabstände können jedoch in Abhängigkeit von den jeweiligen Bedingungen ebenfalls vorteilhaft sein.

Die Entnahme von etwa 0,3 bis etwa 0,6 ml Cerebrospinalflüssigkeit pro Entnahmevorgang stellt eine für das Schaf vergleichsweise geringe Belastung dar und erlaubt darüber hinaus, daß das Cerebrospinalflüssigkeitsreservoir im betreffenden Cerebralventrikel nicht übermäßig strapaziert wird, was ansonsten ggf. zu einer Verfälschung der Werte betreffend die Bioverfügbarkeit und/oder Blut/Hirnschrankengängigkeit der jeweiligen chemischen Verbindung und/oder Kombination chemischer Verbindungen führen würde. Andererseits ist die genannte Probengröße umfangreich genug, um die entsprechenden Analysen auf die chemische Verbindung und/oder Kombination chemischer Verbindungen sowie eventueller Metabolite vornehmen zu können.

Indem die Vorrichtung zur Entnahme von Cerebrospinalflüssigkeit aus einem Material hergestellt ist, das aus der Gruppe ausgewählt ist, die rostfreien Stahl und Teflon umfaßt, wird gewährleistet, daß durch die Insertion der Vorrichtung in die im Cerebralventrikel vorhandene Cerebrospinalflüssigkeit diese hinsichtlich ihrer chemischen Konsistenz nicht beeinträchtigt wird und darüber hinaus die für die Bestimmung der Bioverfügbarkeit und/oder Blut/Hirnschrankengängigkeit erforderliche nachfolgende Analyse keinerlei durch das Probenahmesystem bedingten Restriktionen unterworfen wird.

Darüber hinaus sind die genannten Materialien biokompatibel und infolgedessen ist gewährleistet, daß es zu keinen stärkeren Irritationen des Gewebes kommt, was besonders bei einem längeren Verbleib der Vorrichtung zur Entnahme von Cerebrospinalflüssigkeit im Cerebralventrikel von Bedeutung ist.

Die Vorrichtung zur Entnahme von Cerebrospinalflüssigkeit kann in vorteilhafter Weise ausgewählt sein aus der Gruppe, die kommerziell erhältliche Injektions- und Punktionskanülen umfaßt. Somit wird gewährleistet, daß das erfindungsgemäße Verfahren unter Verwendung eines vergleichsweise leicht zu beschaffenden und kostengünstigen Instrumentariums durchgeführt werden kann, das darüber hinaus in der erforderlichen Vielfalt erhältlich ist.

Wird, wie in einer Alternative des erfindungsgemäßen Verfahrens vorgesehen, nach der letzten Entnahme von Cerebrospinalflüssigkeit die Vorrichtung zu deren Entnahme aus dem Cerebralventrikel entfernt, kann das Tier, ggf. nach geeignetem Verschluß der Trepanationsöffnung, wieder unter den üblichen Bedingungen gehalten werden, was besonders dann von Vorteil ist, wenn Langzeituntersuchungen angestellt werden sollen. Die entsprechende Entnahme ist besonders dann von Vorteil, wenn das Tier wieder in einer Herde gehalten werden soll, wird doch dadurch vermieden, daß infolge von Sozialkontakten vermittels der Entnahmevorrichtung Hirnstrukturen beschädigt werden.

Umgekehrt kann auch nach der letzten Entnahme von Cerebrospinalflüssigkeit die Vorrichtung zur Entnahme derselben im Cerebralventrikel verbleiben. Dadurch wird es möglich, über längere Zeiträume das erfindungsgemäße Verfahren, sei es nun mit der identischen chemischen Verbindung oder Kombination von chemischen Verbindungen oder einer anderen zu wiederholen und damit weitergehende Auskünfte betreffend die Bioverfügbarkeit und/oder Blut/Hirnschrankenverfügbarkeit der entsprechenden Verbindungen zu erhalten.

Nach dem erfindungsgemäßen Verfahren erfolgt die Bestimmung der Bioverfügbarkeit und/oder Blut/Hirnschrankengängigkeit typischerweise an einem nicht betäubten Schaf, wenngleich das Tier unter bestimmten Umständen auch während des Verfahrens betäubt sein kann. Um jedoch die genannten Werte unter weitestgehend normalen physiologischen Bedingungen, d.h. an einem aktiven, nicht betäubten Organismus, ermitteln zu können, wird das Tier im erfindungsgemäßen Verfahren normalerweise nicht betäubt sein. Davon ausgenommen kann der Zeitpunkt der Eröffnung der Schädeldecke sein, während der das Tier betäubt ist. Unter den letztgenannten Bedingungen wird den Empfehlungen der "Good Laboratory Practice" und der "Good Clinical Practice" entsprochen und es ist vergleichsweise einfacher und für den tierischen Organismus auch weniger belastend, die Vorrichtung zur Entnahme von Cerebrospinalflüssigkeit in den relevanten Gehirnventrikel zu inserieren.

Um den, wie bereits mehrfach erwähnten physiologischen Bedingungen möglichst nahe zu kommen, sieht das erfindungsgemäße Verfahren vor, daß das Schaf nach Applikation der chemischen Verbindung(en) frei laufen gelassen wird. Damit werden Bedingungen realisiert, die aus dem Grund besonders interessant sind, weil das Tier seinem natürlichen Bewegungsdrang ohne irgendwelchen Einschränkungen nachgehen kann, und Streßfaktoren infolge beispielsweise einer bei anderen Verfahren unter Umständen erforderlichen Fixierung vermieden werden, was mit Blick auf die Tatsache, daß Streßzustände eine Änderung der Werte betreffend die Bioverfügbarkeit und/oder Blut/Hirn- Schrankengängigkeit von chemischen Verbindungen grundsätzlich zu beeinflussen scheinen, für eine Vielzahl von Fragestellungen sehr aufschlußreich sein kann.

Für die Realisierung der Vorteile des erfindungsgemäßen Verfahrens bedarf es keiner speziellen Schafrasse. Schafe aus Kreuzungsrassen sind - auch - geeignet.

Die genannten Vorteile des erfindungsgemäßen Verfahrens ergeben sich unabhängig von der Art und Weise, wie die chemische Verbindung oder Kombination von chemischen Verbindungen appliziert wird. Grundsätzlich sind alle Möglichkeiten der Applikation derartiger Verbindungen, die in der Fachwelt bekannt sind, wobei der Begriff der Fachwelt hier nicht nur auf den Bereich Pharmakologie und Toxikologie beschränkt sein soll, im Rahmen des erfindungsgemäßen Verfahrens anwendbar. Dies bedeutet, daß intramuskuläre, intravenöse, intraarterielle, intraperitoneale und subkutane Injektion ebenso vorgesehen ist, wie die nasale Applikation oder die topische Applikation der chemischen Verbindung oder Kombination von chemischen Verbindungen. Gleiches gilt auch für jegliche Form der Applikation auf Oberflächen oder der Applikation über den Magen-Darm-Trakt. Auch bestehen keinerlei Einschränkungen betreffend die galenischen Darreichungsformen, in denen die chemische Verbindung oder Kombination von chemischen Verbindungen dem Tier appliziert werden soll.

Diese umfassen unter anderem Injektionslösungen, Tabletten, Dragees, Suppositorien, Tropfen, Augentropfen, Sprays, Aerosole, Gele, Salben, Cremes und Pflaster.

Es ist im Rahmen der vorliegenden Erfindung, daß die Bestimmung der Konzentration der chemischen Verbindung(en) in der dem Cerebralventrikel des Schafes entnommenen Cerebrospinalflüssigkeit auch die Bestimmung möglicher Metabolite umfaßt, sowie, daß die Bestimmung der besagten Metabolite anstelle der Bestimmung der chemischen Verbindung(en) erfolgt, mithin hierin unter der Bezeichnung chemische Verbindung auch Metabolite derselben verstanden werden.

Weitere Vorteile und Merkmale ergeben sich aus der beiliegenden Beschreibung eines Ausführungsbeispiels sowie der beigefügten Figuren, wobei
Fig. 1 einen schematischen Längsschnitt an der sagittalen Nullebene des Hypothalamus des Schafes in stereotaktischen Koordinaten, wobei AC für Commissura anterior, FX für Fornix, CM für Corpus mamillaris, TH für Thalamus, RI für Recessus infundibularis und vIII für Ventriculus tertius steht; und
Fig. 2 den Konzentrationsverlauf von Progesteron im Blutplasma und in der Cerebrospinalflüssigkeit eines Schafes in Abhängigkeit von der Zeit zeigt.

### Beispiel:

Ein weibliches Schaf mit einer Masse von ca. 45 kg wurde einen Tag vor der Implantation der Vorrichtung zu Entnahme von Cerebrospinalflüssigkeit von der Herde getrennt in einer eigenen Box gehalten und erhielt kein Futter. Im Rahmen der Vorbereitung des Eingriffes wurden dem Tier Dehydrobenzperidol intramuskulär in einer Dosis von 1,5 mg/kg und Atropin in einer Dosis von insgesamt 11 mg intramuskulär verabreicht. Daraufhin wurde eine Kanüle in die Vena jugularis ext. mit einem Innendurchmesser von 1,6 mm eingeführt und fixiert. Thiopental wurde in einer Dosis von 15 mg/kg zur Betäubung durch die Kanüle verabreicht. Anschließend wurde dem Tier Halothan anfangs in einer Konzentration von 2 % für ca. 5 Minuten verabreicht. Danach wurde die Konzentration auf 1,5 bis 1,25 % entsprechend der Intensität der Narkose während der Operation eingestellt. Die Trägermischung bestand aus Sauerstoff und N₂O und wurde auf ein Verhältnis von 1:1 bis 7:3 eingestellt. Während der gesamten Anästhesie wurde Atropin in einer Dosis von 0,5 mg/kg in 20-minütigem Abstand intravenös durch die Kanüle verabreicht.

Nach Resektion der Ohrmuscheln wurde der Kopf des Schafes in den Rahmen der stereotaktischen Vorrichtung plaziert und mittels Ohrhalter, die in die äußeren Gehörgänge inseriert wurden, fixiert. Danach wurde der Kopf in die horizontale Ebene der Vorrichtung gedreht, so daß die Augenhalter an die unteren Orbitalfissuren angelehnt waren. In dieser Position wurde der Kopf zusätzlich durch Kieferhalter fixiert, die den harten Gaumen an der Stelle der ersten Prämolaren unterstützten. Der Kopf wurde im Bereich des Schädelgewölbes geschoren und desinfiziert. Der Einschnitt in die Haut, etwa 4-6 cm lang, wurde in der medialen Ebene vorgenommen. Subkutanes Gewebe und Muskeln wurden mittels eines stumpfen Instrumentes entfernt.

Nach Einschnitt des Periosteums, ebenfalls in der medialen Ebene des Kopfes, wurden die Schädelknochen in einem Bereich freigelegt, der rostral durch die Sutura coronoidea und Sutura sagittalis und kaudal durch die Sutura lambdoidea begrenzt ist.

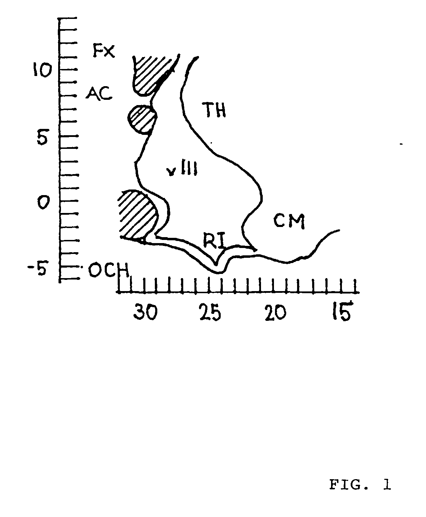

Die intraventrikuläre Kanüle wurde auf den Nullpunkt, entsprechend der Mitte der die beiden äußeren Gehörgänge verbindenden Achse, auf der Zentrierplatte der Vorrichtung ausgerichtet. Die solchermaßen innerhalb der Nullebenen der Vorrichtung zentrierte Kanüle wurde mittels des Schiebers der Vorrichtung über den Schädel des Schafes bewegt und die Position des Bregma-Punktes und des Lambda-Punktes mit ihren antero-posterior Bezugskoordinaten verglichen. Die antero-posterior Koordinaten können von diesen Punkten für das Gebiet des dritten Cerebralventrikels abgeleitet werden. Hierzu wurde unterstützend das in Figur 1 dargestellt Diagramm der hypothalamischen Region des Schafes in stereotaktischen Koordinaten herangezogen.

Anschließend wurde eine Öffnung mit einer Größe von 10x15 mm in den Schädel unter Verwendung eines elektrischen Mikrobohrers am solchermaßen bestimmten Punkt gebohrt und die Dura mater von außen gereinigt, um Knochensplitter zu entfernen. Bohrer aus rostfreiem Stahl, wie sie in der Zahnmedizin verwendet werden, mit einem Durchmesser von 3,3 mm wurden für das Bohren der Öffnung verwendet. Danach wurden Vertiefungen zum Versenken von drei Schrauben aus rostfreiem Stahl in die Schädelknochen in der Umgebung der Trepanationsöffnung gebohrt, zwei rostral der Öffnung und eine kaudal.

Die intraventrikuläre Kanüle, die mit einem Mandrin und einer äußeren Führung ausgestattet ist, wurde vertikal an den ausgewählten antero-posterioren und sagittalen Koordinaten abgesenkt, bis sie die Dura mater berührte. Die Dura mater wurde durch die scharfe Spitze der Kanüle perforiert und weiter durch das Vorderhirn abgesenkt bis zu dem Punkt, an dem die Kanüle den Boden der Schädelhöhle erreichte. Mittels eines Vergleichs der Koordinaten des Schädelbodens mit den Horizontalkoordinaten im Diagramm des dritten Cerebralventrikels wurden die Horizontalkoordinaten der Kanüle bestimmt und damit auch die Stelle der Punktierung des dritten Cerebralventrikels.

Wenn der dritte Cerebralventrikel erfolgreich punktiert wurde, trat, nachdem der Mandrin aus der Kanüle entfernt worden war, unter Pulsieren Cerebrospinalflüssigkeit aus, wobei das Pulsieren synchron zu den Herzschlägen erfolgte. Danach wurde die Kanüle in der Trepanationsöffnung mittels Acrylatharz fixiert.

Nach Entfernen der Halter der stereotaktischen Vorrichtung wurde der vorstehende Teil der Kanüle durch einen Plastikzylinder geschützt, der in das Acrylatharz eingesenkt und mittels Schrauben im Schädel verankert wurde. Die Hautbereiche wurden mit Nylon zusammengenäht und der Operationspunkt mit einem leichten Verband bedeckt. Danach wurde die Narkose dadurch beendet, daß die weitere Zufuhr von Halothan abgestellt wurde. Nach den ersten Zeichen, daß das Tier aus der Narkose erwachte, wurde die intravenöse Kanüle entfernt und, nachdem das Tier wieder auf den Beinen stand, es in eine geeignete Box gebracht. Nach Abschluß der Operation wurde das Tier für sieben Tage mit intramuskulär verabreichten Antibiotika und Hydrocortison versorgt. Die Genesung erfolgte in allen Fällen ohne außerordentliche Vorkommnisse.

Die Punktierung des dritten Cerebralventrikels und das SammeIn von Cerebrospinalflüssigkeit mit einer vor kurzem inserierten permanenten Kanüle wurde an einem stehenden, nicht anaesthesierten Tier vorgenommen.

Nachdem der Mandrin aus der Kanülenführung entfernt worden war, wurde der Auslaß der Dauerkanüle mit 70 %-igem Alkohol desinfiziert und mit einem vorab sterilisierten Tupfer ausgewischt. Die Punktierungskanüle wurde vorab sterilisiert.

Die transparente Teflonkanüle mit einer Länge von 25 cm wurde mittels der Kanülenführung an die Stelle geführt, die nach einer erfolgreichen stereotaktischen Operation markiert worden war.

Nachdem die Teflonkanüle unterhalb des Spiegels des dritten Cerebralventrikels geneigt worden war, floß Cerebrospinalflüssigkeit langsam heraus. Somit war die Möglichkeit gegeben, Cerebrospinalflüssigkeit zu sammeln.

Das solchermaßen vorbereitete Schaf erhielt eine intramuskuläre Injektion von Progesteron (handelsübliches Präparat in öliger Lösung) mit einer Dosis von 15 mg pro Tier. Vor der Injektion sowie 5, 15, 30, 60, 90, 120, 150, 180 und 360 Minuten nach der Injektion wurden Blutproben aus der V. jugularis entnommen.

Zu den gleichen Zeitpunkten wurden jeweils 0,3 ml Cerebrospinalflüssigkeit aus dem dritten Ventrikel genommen. In der Zeit zwischen den einzelnen Sammel- bzw. Entnahmevorgängen wurde die Punktierungskanüle in 70 % Ethanol eingelegt. Nach der letzten Entnahme von Cerebrospinalflüssigkeit aus dem dritten Ventrikel wurde die Kanüle mit dem Mandrin verschlossen.

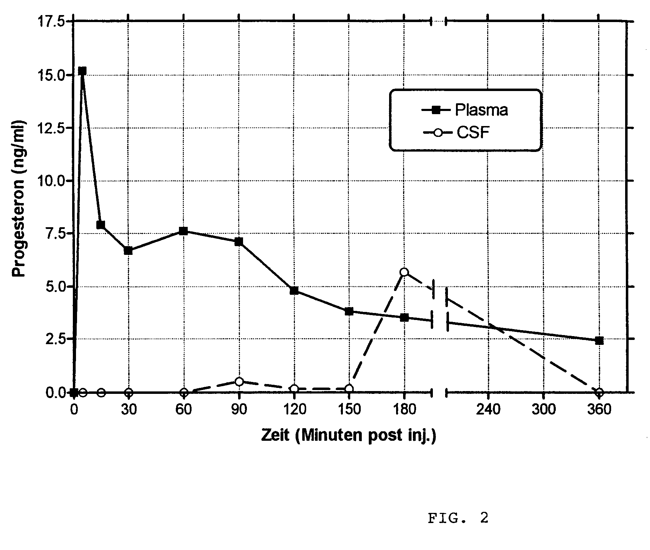

Die Konzentration des Progesterons in der Cerebrospinalflüssigkeit sowie im Blut wurde mittels Radioimmunassay spezifisch bestimmt. Die Ergebnisse sind in Fig. 2 dargestellt.

In Fig. 2 zeigt sich die prinzipiell bekannte Pharmakokinetik des Progesterons. In der Cerebrospinalflüssigkeit kann Progesteron erst 90 Minuten nach der Injektion in geringen Konzentrationen nachgewiesen werden. Das Maximum der Konzentration wird 180 Minuten nach Injektion erreicht.

Mit diesen Ergebnissen konnte erstmals gezeigt werden, daß das Steroidhormon Progesteron nach systemischer Applikation in der Lage ist, durch die Blut/Hirn-Schranke zu permeieren. Hieraus kann geschlossen werden, daß pharmakodynamische Effekte des Progesterons im Zentralnervensystem durch Bindung an entsprechende Rezeptoren ausgelöst werden können.

Die vorgenannte vergleichende Untersuchung der Pharmakokinetik von Progesteron im Blut und im Liquor nach intramuskulärer Injektion eines Progesteronpräparates belegt die Eignung und Funktionsfähigkeit des Models bzw. des vorgestellten Verfahrens.

Die in der vorstehenden Beschreibung sowie in den Ansprüchen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Verfahren zur Bestimmung der Bioverfügbarkeit und/oder Blut/Hirnschrankengängigkeit einer chemischen Verbindung oder Kombination von chemischen Verbindungen, dadurch gekennzeichnet, daß
a) eine Vorrichtung zur Entnahme von Cerebrospinalflüssigkeit in einen Cerebralventrikel eines Schafes inseriert wird;
b) die chemische Verbindung oder Kombination von chemischen Verbindungen dem Schaf appliziert wird;
c) Cerebrospinalflüssigkeit aus dem Cerebralventrikel entnommen wird; und
d) die Konzentration der chemischen Verbindung(en) in der dem Cerebralventrikel des Schafes entnommenen Cerebrospinalflüssigkeit bestimmt wird.

2. Verfahren nach Anspruch 1, dadurch gekennzeichnet, daß auch vor Schritt b) Cerebrospinalflüssigkeit aus dem Cerebralventrikel entnommen wird.

3. Verfahren nach einem der Ansprüche 1 und 2, dadurch gekennzeichnet, daß der Cerebralventrikel der dritte Cerebralventrikel ist.

4. Verfahren nach einem der Ansprüche 1 bis 3, dadurch gekennzeichnet, daß die Vorrichtung zur Entnahme von Cerebrospinalflüssigkeit eine Kanüle ist.

5. Verfahren nach einem der Ansprüche 1 bis 4, dadurch gekennzeichnet, daß die Vorrichtung zur Entnahme von Cerebrospinalflüssigkeit stereotaktisch in den Cerebralventrikel inseriert wird.

6. Verfahren nach Anspruch 5, dadurch gekennzeichnet, daß die stereotaktische Insertion auf der Basis des Knochenreferenzsystems erfolgt.

7. Verfahren nach Anspruch 6, dadurch gekennzeichnet, daß die stereotaktische Insertion zusätzlich auf der Basis geeigneter stereotaktischer Diagramme erfolgt.

8. Verfahren nach einem der Ansprüche 1 bis 7, dadurch gekennzeichnet, daß die Schädeldecke an der Stelle, an der die Vorrichtung zur Entnahme von Cerebrospinalflüssigkeit inseriert werden soll, mittels gezielter Trepanation eröffnet wird.

9. Verfahren nach einem der Ansprüche 1 bis 8, dadurch gekennzeichnet, daß die Dura mater durch die Spitze der Vorrichtung zur Entnahme von Cerebrospinalflüssigkeit perforiert wird.

10. Verfahren nach einem der Ansprüche 1 bis 9, dadurch gekennzeichnet, daß die Vorrichtung zur Entnahme von Cerebrospinalflüssigkeit aus zwei konzentrischen Röhren besteht, wobei die äußere Röhre als Führung dient und die innere Röhre beweglich und für die Entnahme von Cerebrospinalflüssigkeit aus dem Cerebralventrikel bestimmt ist.

11. Verfahren nach Anspruch 10, dadurch gekennzeichnet, daß die äußere Röhre der Kanüle in der Trepanationsöffnung fixiert wird.

12. Verfahren nach Anspruch 11, dadurch gekennzeichnet, daß die äußere Röhre der Kanüle mit Acrylatharz fixiert wird.

13. Verfahren nach einen der Ansprüche 1 bis 12, dadurch gekennzeichnet, daß die chemische Verbindung oder Kombination von chemischen Verbindungen mindestens eine pharmakologisch wirksame Substanz umfaßt.

14. Verfahren nach einem der Ansprüche 1 bis 13, dadurch gekennzeichnet, daß die chemische Verbindung oder Kombination von chemischen Verbindungen eine galenische Darreichungsform umfaßt.

15. Verfahren nach einem der Ansprüche 1 bis 14, dadurch gekennzeichnet, daß die chemische Verbindung oder Kombination von chemischen Verbindungen einmalig appliziert wird.

16. Verfahren nach einem der Ansprüche 1 bis 14, dadurch gekennzeichnet, daß die chemische Verbindung oder Kombination von chemischen Verbindungen mehrfach appliziert wird.

17. Verfahren nach einem der Ansprüche 1 bis 16, dadurch gekennzeichnet, daß die chemische Verbindung oder Kombination von chemischen Verbindungen als Depot appliziert wird.

18. Verfahren nach einem der Ansprüche 1 bis 17, dadurch gekennzeichnet, daß Cerebrospinalflüssigkeit spontan aus der Vorrichtung zur Entnahme von Cerebrospinalflüssigkeit austritt.

19. Verfahren nach Anspruch 18, dadurch gekennzeichnet, daß Cerebrospinalflüssigkeit spontan aus der Vorrichtung zur Entnahme von Cerebrospinalflüssigkeit nach Entnahme eines Verschlußmittels aus der besagten Vorrichtung austritt.

20. Verfahren nach einem der Ansprüche 1 bis 19, dadurch gekennzeichnet, daß die Cerebrospinalflüssigkeit abgesaugt wird.

21. Verfahren nach Anspruch 20, dadurch gekennzeichnet, daß die Cerebrospinalflüssigkeit mit einer Spritze abgesaugt wird.

22. Verfahren nach einem der Ansprüche 1 bis 21, dadurch gekennzeichnet, daß in zeitlichen Abständen mehrfach hintereinander Cerebrospinalflüssigkeit entnommen wird.

23. Verfahren nach Anspruch 22, dadurch gekennzeichnet, daß die Entnahme von Cerebrospinalflüssigkeit in Abständen von etwa 15 Minuten erfolgt.

24. Verfahren nach einem der Ansprüche 1 bis 23, dadurch gekennzeichnet, daß pro Entnahme etwa 0,3 bis etwa 0,6 ml Cerebrospinalflüssigkeit entnommen werden.

25. Verfahren nach einen der Ansprüche 1 bis 24, dadurch gekennzeichnet, daß die Vorrichtung zur Entnahme von Cerebrospinalflüssigkeit aus einem Material hergestellt ist, das aus der Gruppe ausgewählt ist, die rostfreien Stahl und Teflon umfaßt.

26. Verfahren nach einem der Ansprüche 1 bis 25, dadurch gekennzeichnet, daß die Vorrichtung zur Entnahme von Cerebrospinalflüssigkeit aus der Gruppe ausgewählt ist, die kommerziell erhältliche Injektions- und Punktionskanülen umfaßt.

27. Verfahren nach einem der Ansprüche 1 bis 26, dadurch gekennzeichnet, daß nach der letzten Entnahme von Cerebrospinalflüssigkeit die Vorrichtung zur Entnahme von Cerebrospinalflüssigkeit aus dem Cerebralventrikel entfernt wird.

28. Verfahren nach einem der Ansprüche 1 bis 26, dadurch gekennzeichnet, daß nach der letzten Entnahme von Cerebrospinalflüssigkeit die Vorrichtung zur Entnahme von Cerebrospinalflüssigkeit im Cerebralventrikel verbleibt.

29. Verfahren nach einem der Ansprüche 1 bis 28, dadurch gekennzeichnet, daß das Schaf während der Öffnung der Schädeldecke betäubt ist.

30. Verfahren nach einem der vorangehenden Ansprüche, dadurch gekennzeichnet, daß das Schaf nach Applikation der chemischen Verbindung(en) frei laufen gelassen wird.
